# EUROPEAN PATENT APPLICATION

(11) **EP 1 469 084 A2**
(43) Date of publication of application: **20.10.2004**
(21) Application number: 04014565.8
(22) Date of filing: 30.10.2001
(51) Int. Cl.: C12Q 1/68

(54) **Polymorphisms in the human KDR gene**

(30) Priority: 03.11.2000 GB 0026851; 19.04.2001 GB 0109602
(62) Divisional of application: 01309193.9
(71) Applicant: AstraZeneca AB, 151 85 Södertälje (SE)
(72) Inventor: Smith, John Craig, Macclesfield, Cheshire SK10 4TG (GB)

(57) **Abstract**

This invention relates to polymorphisms in the human KDR gene and corresponding novel allelic polypeptides encoded thereby. In particular, the invention relates to two polymorphisms in the coding sequence of the KDR gene, each of which lead to an amino acid change in the sequence of expressed protein; three single nucleotide polymorphisms (SNPs) in the promoter region; one SNP in the 5' UTR and seven SNPs in intronic sequence. The invention also relates to methods and materials for analysing allelic variation in the KDR gene, and to the use of KDR polymorphism in treatment of KDR ligand (Vascular Endothelial Growth Factor VEGF)-mediated diseases such as cancer and various inflammatory diseases.

SEQUENCE LISTING

<110> ASTRAZENECA AB

<120> CHEMICAL COMPOUNDS

<130> LDG/100189

<140>
<141>

<150> 0026851.6
<151> 2000-11-03

<160> 25

<170> PatentIn Ver. 2.1

<210> 1
<211> 519
<212> DNA
<213> Human

<400> 1
cctaaggata tcttggctgg aagctctgct ctgaaaaggg gcatggccaa actttcacta 60
gggctcttcg ttggggagca cgatggacaa aagccttctt ggggctaggc aggtcacttc 120
aaacttggag ccgccaaata ttttgggaaa tagcgggaat gctggcgaac tgggcaagtg 180
cgttttctga ttaagagcaa ccagattcag ctttttaaac tacaattata ctggccaaac 240
aaaataccct tatacaaaaa ccaaaactac tggcaggagt cgctgccagc ttgcgacccg 300
gcatacttgg ctgagtatcc gcttctccct tgtggctcca aactgctgca gattctcggc 360
cacttcagac gcgcgcgatg gcgaagaggg tcctgcactt tgacgcgcct ggtgagggag 420
cggtgctctt cgcagcgctc ctggtgatgc tccccaaatt tcggggaccg gcaagcgatt 480
aaatcttgga gttgctcagc gcccgttacc gagtacttt 519


<210> 2
<211> 503
<212> DNA
<213> Human

<400> 2
ctgttatgac acagcttgtg aacttttact gagaatggtg aaaagtaaat tcccagtttt 60
atacaatgaa ttgctgaaga ggccttttaa agtatagagt atgcattgtt tatggaaggt 120
gtttcctatt aggtctaact cagtggcaac tacattcatt tatttaattt gtttctaggt 180
ttgcctagtg tttctcttga tctgcccagg ctcagcatac aaaaagacat acttacaatt 240
aaggctaata caactcttca aattacttgc aggtaaggat tcattctaga tctagatttc 300
ttgtgttaag taactgattg tttattgagt ggaaataatt tccagtagag cagaattata 360
atagagcttg tagtaattgt tcataagtgg tgaggtttct aagaactgat gtaataatgg 420
aaaatgagaa gaattttctc tcaaaaattc tgtacaattt tgctggtgtt tttatactat 480
tctctgccaa catgcataca cac 503


<210> 3
<211> 397
<212> DNA
<213> Human

<400> 3
caagtattgg tgaggcctgc agggaaaaac aatgtggcat gttttaaagt tgcattactt 60
taaaaaataa atctgtgcaa agttataggc ttatttgctc tctcatgttc tgtttttca 120
atttacttgc tctagggtat aggatttatg atgtggttct gagtccgtct catggaattg 180
aactatctgt tggagaaaag cttgtcttaa attgtacagc aagaactgaa ctaaatgtgg 240
ggattgactt caactgggaa tacccttctt cgaaggtaac gctaatgatt caaagccaga 300
cctccaaata cttacataat aagccccagt gaagtttgct tgagagatag gggcctcttt 360
ggccagataa aatgtaagag ccttaaacac acacaca 397


<210> 4

<211> 453
<212> DNA
<213> Human

<400> 4
aaagttctgg tgtccctgtt tttagcatta aataatgttt accaaagccc agatttaatt 60
ctgtgtgtta ctagaagtta ttgggtaatg ttatatgctg tgctttggaa gttcagtcaa 120
ctcttttttt cagcatcagc ataagaaact tgtaaaccga gacctaaaaa cccagtctgg 180
gagtgagatg aagaaatttt tgagcacctt aactatagat ggtgtaaccc ggagtgacca 240
aggattgtac acctgtgcag catccagtgg gctgatgacc aagaagaaca gcacatttgt 300
cagggtccat ggtaagctat ggtcttggaa attattctgt gccttgacaa gtgagataat 360
ttaaataaat ttaggtcact tagtgattcc tattttgttc attcagaaga tagtttctag 420
tttttcttgt tagggaggcc acatgaccta gag 453

<210> 5
<211> 377
<212> DNA
<213> Human

<400> 5
tttcgtgtga gagcaaatcc tttgactata ctaattcctg agaatttttt ttcataggta 60
taaaaatgga ataccccttg agtccaatca cacaattaaa gcggggcatg tactgacgat 120
tatggaagtg agtgaaagag acacaggaaa ttacactgtc atccttacca atcccatttc 180
aaaggagaag cagagccatg tggtctctct ggttgtgtat ggtgagtcca ttcaattttc 240
ctctctgccc aagatttatt atgatacatt gtcttccaaa tcagccaaac caccgttcct 300
ctgcctcctg ctgcttcact catatcatgg ctgggcctgc gtacaaaagt catctggcgt 360
ggtgaagctg aagtgaa 377

<210> 6
<211> 499
<212> DNA
<213> Human

<400> 6
cgccaagtct tgtaccatgg taggctgcgt tggaagttat ttctaagaac agaataacca 60
aagtctgaat cttttcctta ctcttgactc taattaaaga aaaattaaat cataatatgc 120
gctgttatct ctttcttata gccaagctgt ctcagtgaca aacccatacc cttgtgaaga 180
atggagaagt gtggaggact tccagggagg aaataaaatt gaagttaata aaaatcaatt 240
tgctctaatt gaaggaaaaa acaaagtgag tttgaagttt taaaatttga aaatctctct 300
ctctttaatg gaaggatggt acaataatat gtgaggcata ttggagatta ataatcaaat 360
agtctggatg attaaataga gcgtattaag tcactttgaa aataccattg acttttagca 420
gtaccattaa cttattaata gcttatcaga gaaaaataaa aacatctatg acattaaatc 480
tatgcatctg tgtagggtg 499

<210> 7
<211> 357
<212> DNA
<213> Human

<400> 7
ttgatgtcct ccttgtctgc tttttagtag tttcaattat ctccatggtt tactacattt 60
taaaggttgt aaacttttaa agactcattt tgtattcaag gagtttgttt gttcctttgc 120
ttttttatag accaaagggg cacgattccg tcaagggaaa gactacgttg gagcaatccc 180
tgtggatctg aaacggcgct tggacagcat caccagtagc cagagctcag ccagctctgg 240
atttgtggag gagaagtccc tcagtgatgt agaagaagag gaaggtactg gctagtgctt 300
cctgcatgct atggcatgct cttgtcagag cagacagggt gatagggtgt tacaagg 357

<210> 8
<211> 31
<212> DNA
<213> Human

<400> 8
ttaactatag atggtataac ccggagtgac c 31

<210> 9
<211> 31
<212> DNA
<213> Human

<400> 9
tctttcttat agccatgctg tctcagtgac a                                    31


<210> 10
<211> 101
<212> DNA
<213> Human

<400> 10
agtctgggag tgagatgaag aaatttttga gcaccttaac tatagatggt rtaacccgga 60
gtgaccaagg attgtacacc tgtgcagcat ccagtgggct g                        101


<210> 11
<211> 104
<212> DNA
<213> Human

<400> 11
taaagaaaaa ttaaatcata atatgcgctg ttatctcttt cttatagcca wgctgtctca 60
gtgacaaacc catacccttg tgaagaatgg agaagtgtgg agga                     104


<210> 12
<211> 102
<212> DNA
<213> Human

<400> 12
aggtcacttc aaacttggag ccgccaaata ttttgggaaa tagcgggaat gytggcgaac 60
tgggcaagtg cgttttctga ttaagagcaa ccagattcag ct                       102


<210> 13
<211> 100
<212> DNA
<213> Human

<400> 13
gcgcgatggc gaagagggtc ctgcactttg acgcgcctgg tgagggagcg stgctcttcg 60
cagcgctcct ggtgatgctc cccaaatttc ggggaccggc                          100


<210> 14
<211> 101
<212> DNA
<213> Human

<400> 14
ggtgagggag cggtgctctt cgcagcgctc ctggtgatgc tccccaaatt ycggggaccg 60
gcaagcgatt aaatcttgga gttgctcagc gcccgttacc g                        101


<210> 15
<211> 101
<212> DNA
<213> Human

<400> 15
gatttcttgt gttaagtaac tgattgttta ttgagtggaa ataatttcca rtagagcaga 60

attataatag agcttgtagt aattgttcat aagtggtgag g 101

<210> 16
<211> 101
<212> DNA
<213> Human

<400> 16
aaaaaataaa tctgtgcaaa gttataggct tatttgctct ctcatgttct rttttttcaa 60
tttacttgct ctagggtata ggatttatga tgtggttctg a 101

<210> 17
<211> 101
<212> DNA
<213> Human

<400> 17
acgctaatga ttcaaagcca gacctccaaa tacttacata ataagcccca rtgaagtttg 60
cttgagagat aggggcctct ttggccagat aaaatgtaag a 101

<210> 18
<211> 89
<212> DNA
<213> Human

<400> 18
accaccgttc ctctgcctcc tgctgcttca ctcatatcat ggctgggcct rcgtacaaaa 60
gtcatctggc gtggtgaagc tgaagtgaa 89

<210> 19
<211> 101
<212> DNA
<213> Human

<400> 19
aataaccaaa gtctgaatct tttccttact cttgactcta attaaagaaa mattaaatca 60
taatatgcgc tgttatctct ttcttatagc caagctgtct c 101

<210> 20
<211> 82
<212> DNA
<213> Human

<400> 20
ttgatgtcct ccttgtctgc tttttagtag tktcaattat ctccatggtt tactacattt 60
taaaggttgt aaacttttaa ag 82

<210> 21
<211> 101
<212> DNA
<213> Human

<400> 21
catggtttac tacattttaa aggttgtaaa cttttaaaga ctcattttgt wttcaaggag 60
tttgtttgtt cctttgcttt tttatagacc aaaggggcac g 101

<210> 22
<211> 628
<212> DNA
<213> Human

<400> 22
acttgggcca gagcagtcca gttgtgtgcg aaatgggaat ataaatggct tggggagctg 60
atatagacgc gagatacccg gatgaggggc ggactggccg aacggagagc ccctcctccg 120
atccggcccc gccccgcatg gccccgcctc cgcgctctag agtttcggca ccagctccca 180
ccctgcactg agtcccggga ccccgggaga gcggtcagtg tgtggtcgct gcgtttcctc 240
tgcctgcgcc gggcatcact tgcgcgccgc agaaagtccg tctggcagcc tggatatcct 300
ctcctaccgg cacccgcaga cgcccctgca gccgccggtc ggcgcccggg ctccctagcc 360
ctgtgcgctc aactgtcctg cgctgcgggg tgccgcgagt tccacctccg cgcctccttc 420
tctagacagg cgctgggaga aagaaccggc tcccgagttc tgggcatttc gcccggctcg 480
aggtgcagga tgcagagcaa ggtgctgctg gccgtcgccc tgtggctctg cgtggagacc 540
cgggccgcct ctgtgggtaa ggagcccact ctggaggagg aaggcagaca ggtcgggtga 600
gggcggagag gacctgaaag ccagatct 628

<210> 23
<211> 315
<212> DNA
<213> Human

<400> 23
tagacgcgag atacccggat gaggggcgga ctggccgaac ggagagcccc tcctccgatc 60
cggccccgcc ccgcatggcc ccgcctccgc gctctagagt ttcggcacca gctcccaccc 120
tgcactgagt cccgggaccc cgggagagcg gtcagtgtgt ggtcgctgcg tttcctctgc 180
ctgcgccggg catcacttgc gcgccgcaga aagtccgtct ggcagcctgg atatcctctc 240
ctaccggcac ccgcagacgc ccctgcagcc gccggtcggc gcccgggctc cctagccctg 300
tgcgctcaac tgtcc 315

<210> 24
<211> 51
<212> DNA
<213> Human

<400> 24
tcccgggacc ccgggagagc ggtcartgtg tggtcgctgc gtttcctctg c 51

<210> 25
<211> 20
<212> DNA
<213> Human

<400> 25
tccttggtca ctccgggttg 20

## Description

This invention relates to polymorphisms in the human KDR gene and corresponding novel allelic polypeptides encoded thereby. The invention also relates to methods and materials for analysing allelic variation in the KDR gene, and to the use of KDR polymorphism in treatment of KDR ligand (Vascular Endothelial Growth Factor VEGF)-mediated diseases such as cancer and various inflammatory diseases (Reviewed by Carmeliet and Jain (**2000**) Nature **407**:249-257; Ferrara and Alitalo (1999) Nature Medicine 5:1359-1364) with KDR Inhibitors.

The KDR cDNA (EMBL Accession Number AF035121, 5830 bp) encodes a mature protein of 1356 amino acids. The KDR protein consists of an external domain containing seven immunoglobulin like domains, a transmembrane region and a cytoplasmic region containing a tyrosine kinase domain. In contrast to other members of the receptor tyrosine kinase family, the kinase domain of KDR is in two segments with an intervening sequence of - 70 amino acids. The biology of the VEGF (Vascular Endothelial Growth Factor) receptor family has been reviewed (Neufeld *et al*., (1999) FASEB Journal. **13**:11-22; Zachary (1998) Experimental Nephrology **6**:480-487) and the tyrosine phosphorylation sites have been characterised. Ligand binding sites in the extracellular domain of KDR have been identified (Lu et al, (2000) J Biol Chem **275**:14321-14330). The genomic structure of the human KDR gene has been described (Yin et al (1998) Mammalian Genome. **9**:408-410.). The gene contains 28 exons spanning 45 kb and has been localised to chromosome 4 q11-q12 (Sait et al (1995) Cytogen Cell Genet **70**:145-146; Spritz et al (1994) Genomics **22**:431-436). The genomic sequence of contigs spanning the region containing the KDR gene has been published (EMBL Accession AC021220, 226334 bp).

The location of the polymorphisms can be precisely mapped by reference to published EMBL (or other sequence database) sequence accession numbers (i.e. EMBL Accession Number AC021220 or AF035121), alternatively, the person skilled in the art can precisely identify the location of the polymorphism in the KDR gene simply by provision of flanking sequence adjacent the polymorphism sufficient to unambiguously locate the polymorphism. Provision of 10 or more nucleotides each side of the polymorphism should be sufficient to achieve precise location mapping of the particular polymorphism.

It is thought that KDR (VEGFR2) mediates the mitogenic and angiogenic effects of VEGF in endothelial cells while another VEGF receptor (flt-1) plays an important role in regulating the tissue architecture in developing vasculature. Evidence to support this theory has come from knockout studies in mice (Fong et al., (1995) Nature **376**:66-70; Shalaby et al., (1995) Nature **376:**62-66) and from biochemical studies (Waltenberger et al., (1994) J Biol Chem. **269**:26988-26995). Activation of endothelial cells by VEGF leads to autophosphorylation of KDR and subsequent tyrosine phosphorylation of multiple downstream targets (reviewed by Gingras et al., (2000) Biochem J. **348**:273-280).

VEGF and its receptors are overexpressed in many tumour types and blocking of VEGF function inhibits angiogenesis and suppresses growth of tumours while overexpression of VEGF enhances angiogenesis and tumour growth (Skobe et al., (1997) Nature Medicine **3**:1222-12227). Compounds acting at the KDR receptor are therefore indicated as pharmaceuticals for use in the treatment of a wide variety of tumours (Ferrara and Alitalo (1999) Nature Medicine **5**:1359-1364).

The use of knowledge of polymorphisms to help identify patients most suited to therapy with particular pharmaceutical agents is often termed "pharmacogenetics". Pharmacogenetics can also be used in pharmaceutical research to assist the drug selection process. Polymorphisms are used in mapping the human genome and to elucidate the genetic component of diseases. The reader is directed to the following references for background details on pharmacogenetics and other uses of polymorphism detection: Linder *et al.* (1997), Clinical Chemistry, **43**:254; Marshall (1997), Nature Biotechnology. **15**:1249; International Patent Application WO 97/40462, Spectra Biomedical; and Schafer *et al*, (1998), Nature Biotechnology. **16**:33.

Clinical trials have shown that patient response to treatment with pharmaceuticals is often heterogeneous. Thus there is a need for improved approaches to pharmaceutical agent design and therapy.

Point mutations in polypeptides will be referred to as follows: natural amino acid (using 1 or 3 letter nomenclature), position, new amino acid. For (a hypothetical) example "D25K" or "Asp25Lys" means that at position 25 an aspartic acid (D) has been changed to lysine (K). Multiple mutations in one polypeptide will be shown between square brackets with individual mutations separated by commas. The presence of a particular base at a polymorphism position will be represented by the base following the polymorphism position. For (a hypothetical) example, the presence of adenine at position 300 will be represented as: 300A.

The present invention is based on the discovery of polymorphisms in the KDR gene. In particular, we have found two polymorphisms in the coding sequence of the KDR gene, each of which lead to an amino acid change in the sequence of expressed protein. We have also found three single nucleotide polymorphisms (SNPs) in the promoter region, one SNP in the 5' UTR and seven SNPs in intronic sequence.

As defined herein, the KDR gene includes exon coding sequence, intron sequences intervening the exon sequences and, 3' and 5' untranslated region (3' UTR and 5' UTR) sequences, and the promoter element of the KDR gene upstream of the 5' UTR.

For the avoidance of doubt the location of the two codon change polymorphisms (emboldened; only the unpublished allele illustrated) and nucleic acid sequence immediately flanking said polymorphism sites is as follows: (a) (codon 297) TTAACTATAGATGGTATAACCCGGAGTGACC (SEQ ID No.8) (b) (codon 472) TCTTTCTTATAGCCATGCTGTCTCAGTGACA (SEQ ID No.9) (Codon 297 is encoded by nucleotides 1192-1194 of AF035121, codon 472 is encoded by nucleotides 1717-1719 of AF035121)

According to one aspect of the present invention there is provided a method for the diagnosis or detection of a polymorphism in KDR in a human, which method comprises determining the sequence of the human at at least one polymorphic position and determining the status of the human by reference to polymorphism in KDR. Preferred polymorphic positions are one or more of the following positions:
162, 423, 461 according to SEQ ID NO: 1;
345 according to SEQ ID NO: 2;
112, 329 according to SEQ ID NO: 3;
224 according to SEQ ID NO: 4;
339 according to SEQ ID NO:5;
103,145 according to SEQ ID NO:6;
32, 94 according to SEQ ID NO:7; and,
26 according to SEQ ID NO:24.

According to another aspect of the invention there is provided a method for the diagnosis of a polymorphism in KDR in a human, which method comprises determining the sequence of the nucleic acid of the human at one or more of positions: 162, 423, 461 (each according to SEQ ID NO: 1); 345 (according to SEQ ID NO: 2);112, 329 (each according to SEQ ID NO: 3); 224 (according to SEQ ID NO: 4); 339 (according to SEQ ID NO:5); 103, 145 (each according to SEQ ID NO:6); 32 and 94 (each according to SEQ ID NO:7), and determining the status of the human by reference to polymorphism in the KDR gene.

According to another aspect of the invention there is provided a method for the diagnosis of a polymorphism in KDR in a human, which method comprises determining the sequence of the nucleic acid of the human at one or more of positions: 162, 423, 461 (each according to SEQ ID NO: 1); 345 (according to SEQ ID NO: 2); 112, 329 (each according to SEQ ID NO: 3); 224 (according to SEQ ID NO: 4); 339 (according to SEQ ID NO:5); 103, 145 (each according to SEQ ID NO:6); 32, 94 (each according to SEQ ID NO:7); and 26 (according to SEQ ID NO: 24), and determining the status of the human by reference to polymorphism in the KDR gene.

According to another aspect of the invention there is provided a method for the diagnosis of a polymorphism in KDR in a human, which method comprises determining the sequence of the human at one or more of the following positions:
positions 162, 423 and 461 in the promoter region of the KDR gene (as defined by the position in SEQ ID NO: 1); position 224 (as defined by the position in SEQ ID NO: 4), and 145 (as defined by the position in SEQ ID NO:6) in the coding region of the KDR gene; positions 345 (as defined by the position in SEQ ID NO: 2), 112 and 329 (as defined by the position in SEQ ID NO: 3), 339 (as defined by the position in SEQ ID NO:5), 103 (as defined by the position in SEQ ID NO:6), 32 and 94 (as defined by the position in SEQ ID NO:7) in the intron region of the KDR gene; and codons 297 and 472 in the KDR polypeptide as defined by the position in EMBL Accession No: AF035121,and determining the status of the human by reference to polymorphism in KDR.

According to another aspect of the invention there is provided a method for the diagnosis of a polymorphism in KDR in a human, which method comprises determining the sequence of the human at one or more of the following positions:
positions 162, 423 and 461 in the promoter region of the KDR gene (as defined by the position in SEQ ID NO: 1); position 224 (as defined by the position in SEQ ID NO: 4), and 145 (as defined by the position in SEQ ID NO:6) in the coding region of the KDR gene; positions 345 (as defined by the position in SEQ ID NO: 2), 112 and 329 (as defined by the position in SEQ ID NO: 3), 339 (as defined by the position in SEQ ID NO:5), 103 (as defined by the position in SEQ ID NO:6); 32, 94 (as defined by the position in SEQ ID NO:7) in the intron region of the KDR gene; position 26 in the 5' UTR region of the KDR gene (as defined by the position in SEQ ID NO: 24); and, codons 297 and 472 in the KDR polypeptide as defined by the position in EMBL Accession No: AF035121,and determining the status of the human by reference to polymorphism in KDR.

In a preferred embodiment, the diagnostic method is used to assess the pharmacogenetics of a drug acting on KDR.

The term human includes both a human having or suspected of having a KDR-mediated disease and an asymptomatic human who may be tested for predisposition or susceptibility to such disease. At each position the human may be homozygous for an allele or the human may be a heterozygote.

The term polymorphism includes single nucleotide substitution, nucleotide insertion and nucleotide deletion which in the case of insertion and deletion includes insertion or deletion of one or more nucleotides at a position of a gene and corresponding alterations in expressed protein.

In one embodiment of the invention preferably the method for diagnosis described herein is one in which the polymorphism at position 162 (according to the position in SEQ ID NO: 1) is the presence of T and/or A.

In another embodiment of the invention preferably the method for diagnosis described herein is one in which the polymorphism at position 423 (according to the position in SEQ ID NO: 1) is the presence of C and/or G.

In another embodiment of the invention preferably the method for diagnosis described herein is one in which the polymorphism at position 461 (according to the position in SEQ ID NO: 1) is the presence of T and/or C.

In another embodiment of the invention preferably the method for diagnosis described herein is one in which the polymorphism at position 224 (according to the position in SEQ ID NO: 4) is the presence of G and/or A.

In another embodiment of the invention preferably the method for diagnosis described herein is one in which the polymorphism at position 145 (according to the position in SEQ ID NO:6) is the presence of A and/or T.

In another embodiment of the invention preferably the method for diagnosis described herein is one in which the polymorphism at position 345 (according to the position in SEQ ID NO: 2) is the presence of G and/or A.

In another embodiment of the invention preferably the method for diagnosis described herein is one in which the polymorphism at position 112 (according to the position in SEQ ID NO: 3) is the presence of G and/or A.

In another embodiment of the invention preferably the method for diagnosis described herein is one in which the polymorphism at position 329 (according to the position in SEQ ID NO: 3) is the presence of G and/or A.

In another embodiment of the invention preferably the method for diagnosis described herein is one in which the polymorphism at position 339 (according to the position in SEQ ID NO:5) is the presence of G and/or A.

In another embodiment of the invention preferably the method for diagnosis described herein is one in which the polymorphism at position 103 (according to the position in SEQ ID NO:6) is the presence of A and/or C.

In another embodiment of the invention preferably the method for diagnosis described herein is one in which the polymorphism at position 32 (according to the position in SEQ ID NO: 7) is the presence of T and/or G.

In another embodiment of the invention preferably the method for diagnosis described herein is one in which the polymorphism at position 94 (according to the position in SEQ ID NO:7) is the presence of A and/or T.

In another embodiment of the invention preferably the method for diagnosis described herein is one in which the polymorphism at position 26 (according to the position in SEQ ID NO: 24) is the presence of G and/or A.

As noted above, at each position the human may be homozygous for an allele or the human may be a heterozygote. If the individual is heterozygote the presence of both alternate polymorphisms may be present.

In a preferred embodiment of the invention the method for diagnosis described herein is one in which the polymorphism in the coding region of the KDR gene:
at position 224 (according to the position in SEQ ID NO: 4) the presence of G and/or A;
and, at position 145 (according to the position in SEQ ID NO:6) is the presence of A and/or T.

In another preferred embodiment of the invention the method for diagnosis described herein is one in which the polymorphism in the KDR protein as defined by the position in EMBL Accession No. AF035121 is either Val297Ile or Gln472His.

The nucleic acid sequence method for diagnosis is preferably one which is determined by a method selected from amplification refractory mutation system, restriction fragment length polymorphism and primer extension. The amino acid sequence method for diagnosis is preferably one which is determined by immunological methods such as enzyme linked immunosorbent assay (ELISA).

In another aspect of the invention there is provided a method for the diagnosis or prognosis of VEGF-mediated disease, which method comprises:
i) obtaining sample nucleic acid from an individual,
ii) detecting the presence or absence of a variant nucleotide at one or more of positions: 162, 423, 461 (each according to SEQ ID NO: 1), 345 (according to SEQ ID NO: 2),112, 329 (each according to SEQ ID NO: 3), 224 (according to SEQ ID NO: 4), 339 (according to SEQ ID NO: 5), 103, 145 (each according to SEQ ID NO: 6), 32 and 94 (each according to SEQ ID NO: 7), in the KDR gene; and,
iii) determining the diagnostic or prognostic status of the individual by reference to polymorphism in the KDR gene.

In another aspect of the invention there is provided a method for the diagnosis or prognosis of VEGF-mediated disease, which method comprises:
i) obtaining sample nucleic acid from an individual,
ii) detecting the presence or absence of a variant nucleotide at one or more of positions: 162, 423, 461 (each according to SEQ ID NO: 1), 345 (according to SEQ ID NO: 2),112, 329 (each according to SEQ ID NO: 3), 224 (according to SEQ ID NO: 4), 339 (according to SEQ ID NO: 5), 103, 145 (each according to SEQ ID NO: 6), 32, 94 (each according to SEQ ID NO: 7); 26 (according to SEQ ID NO:24), in the KDR gene; and,
iii) determining the diagnostic or prognostic status of the individual by reference to polymorphism in the KDR gene.

The status of the individual may be determined by reference to allelic variation at any 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more positions using the allelic variants identified herein, optionally in addition to other variants. Haplotype analysis is particularly useful in determining the status of the individual

The test sample of nucleic acid is conveniently a sample of blood, bronchoalveolar lavage fluid, sputum, or other body fluid or tissue obtained from an individual. It will be appreciated that the test sample may equally be a nucleic acid sequence corresponding to the sequence in the test sample, that is to say that all or a part of the region in the sample nucleic acid may firstly be amplified using any convenient technique e.g. PCR, before analysis of allelic variation.

It will be apparent to the person skilled in the art that there are a large number of analytical procedures which may be used to detect the presence or absence of variant nucleotides at one or more polymorphic positions of the invention. In general, the detection of allelic variation requires a mutation discrimination technique, optionally an amplification reaction and optionally a signal generation system. Table 1 lists a number of mutation detection techniques, some based on the PCR. These may be used in combination with a number of signal generation systems, a selection of which is listed in Table 2. Further amplification techniques are listed in Table 3. Many current methods for the detection of allelic variation are reviewed by Nollau *et al*., Clin. Chem. **43**, 1114-1120, 1997; and in standard textbooks, for example "Laboratory Protocols for Mutation Detection", Ed. by U. Landegren, Oxford University Press, 1996 and "PCR", 2^{nd} Edition by Newton & Graham, BIOS Scientific Publishers Limited, 1997.

### Abbreviations:

| | |
|---|---|
| ALEX™ | Amplification refractory mutation system linear extension |
| APEX | Arrayed primer extension |
| ARMS™ | Amplification refractory mutation system |
| b-DNA | Branched DNA |
| bp | base pair |
| CMC | Chemical mismatch cleavage |
| COPS | Competitive oligonucleotide priming system |
| DGGE | Denaturing gradient gel electrophoresis |
| ELISA | Enzyme Linked ImmunoSorbent Assay |

| | |
|---|---|
| FRET | Fluorescence resonance energy transfer |
| LCR | Ligase chain reaction |
| MASDA | Multiple allele specific diagnostic assay |
| NASBA | Nucleic acid sequence based amplification |
| OLA | Oligonucleotide ligation assay |
| PCR | Polymerase chain reaction |
| PTT | Protein truncation test |
| RFLP | Restriction fragment length polymorphism |
| SDA | Strand displacement amplification |
| SNP | Single nucleotide polymorphism |
| SSCP | Single-strand conformation polymorphism analysis |
| SSR | Self sustained replication |
| TGGE | Temperature gradient gel electrophoresis |

### Table 1 - Mutation Detection Techniques

**General:** DNA sequencing, Sequencing by hybridisation
**Scanning:** PTT*, SSCP, DGGE, TGGE, Cleavase, Heteroduplex analysis, CMC, Enzymatic mismatch cleavage
* Note: not useful for detection of promoter polymorphisms.

### Hybridisation Based

Solid phase hybridisation: Dot blots, MASDA, Reverse dot blots, Oligonucleotide arrays (DNA Chips).

Solution phase hybridisation: Taqman™ - US-5210015 & US-5487972 (Hoffmann-La Roche), Molecular Beacons - Tyagi *et al* (1996), Nature Biotechnology, **14**, 303; WO 95/13399 (Public Health Inst., New York)
**Extension Based**: ARMS™, ALEX™ - European Patent No. EP 332435 B 1 (Zeneca Limited), COPS - Gibbs *et al* (1989), Nucleic Acids Research, **17**, 2347.
**Incorporation Based**: Mini-sequencing, APEX
**Restriction Enzyme Based**: RFLP, Restriction site generating PCR
**Ligation Based:** OLA
**Other**: Invader assay

### Table 2 - Signal Generation or Detection Systems

**Fluorescence:** FRET, Fluorescence quenching, Fluorescence polarisation - United Kingdom Patent No. 2228998 (Zeneca Limited)
**Other**: Chemiluminescence, Electrochemiluminescence, Raman, Radioactivity, Colorimetric, Hybridisation protection assay, Mass spectrometry

### Table 3 - Further Amplification Methods

SSR, NASBA, LCR, SDA, b-DNA

### Table 4- Protein variation detection methods

Immunoassay
Immunohistology
Peptide sequencing

Preferred mutation detection techniques include ARMS™, ALEX™, COPS, Taqman, Molecular Beacons, RFLP, and restriction site based PCR and FRET techniques. Immunoassay techniques are known in the art e.g. A Practical Guide to ELISA by D M Kemeny, Pergamon Press 1991; Principles and Practice of Immunoassay, 2^{nd} edition, C P Price & D J Newman, 1997, published by Stockton Press in USA & Canada and by Macmillan Reference in the United Kingdom.

Particularly preferred methods include ARMS™-allele specific amplification, OLA and RFLP based methods. The allele specific amplification technique known in the art as ARMS™ is an especially preferred method.

ARMS™-allele specific amplification (described in European patent No. EP-B-332435, US patent No. 5,595,890 and Newton et al. (Nucleic Acids Research, Vol. 17, p.2503; 1989)), relies on the complementarity of the 3' terminal nucleotide of the primer and its template. The 3' terminal nucleotide of the primer being either complementary or non-complementary to the specific mutation, allele or polymorphism to be detected. There is a selective advantage for primer extension from the primer whose 3' terminal nucleotide complements the base mutation, allele or polymorphism. Those primers which have a 3' terminal mismatch with the template sequence severely inhibit or prevent enzymatic primer extension. Polymerase chain reaction or unidirectional primer extension reactions therefore result in product amplification when the 3' terminal nucleotide of the primer complements that of the template, but not, or at least not efficiently, when the 3' terminal nucleotide does not complement that of the template.

In a further aspect, the diagnostic methods of the invention are used to assess the pharmacogenetics of a drug acting at KDR.

Assays, for example reporter-based assays, may be devised to detect whether one or more of the above polymorphisms affect transcription levels and/or message stability.

Individuals who carry particular allelic variants of the KDR gene may therefore exhibit differences in their ability to regulate protein biosynthesis under different physiological conditions and will display altered abilities to react to different diseases. In addition, differences arising as a result of allelic variation may have a direct effect on the response of an individual to drug therapy. The diagnostic methods of the invention may be useful both to predict the clinical response to such agents and to determine therapeutic dose.

In a further aspect, the diagnostic methods of the invention, are used to assess the predisposition and/or susceptibility of an individual to diseases mediated by VEGF. This may be particularly relevant in the development of cancer and inflammatory disease, and the present invention may be used to recognise individuals who are particularly at risk from developing these conditions.

In a further aspect, the diagnostic methods of the invention are used in the development of new drug therapies which selectively target one or more allelic variants of the KDR gene. Identification of a link between a particular allelic variant and predisposition to disease development or response to drug therapy may have a significant impact on the design of new drugs. Drugs may be designed to regulate the biological activity of variants implicated in the disease process whilst minimising effects on other variants.

In a further diagnostic aspect of the invention the presence or absence of variant nucleotides is detected by reference to the loss or gain of, optionally engineered, sites recognised by restriction enzymes. The person of ordinary skill will be able to design and implement diagnostic procedures based on the detection of restriction fragment length polymorphism due to the loss or gain of one or more of the restriction sites due to the presence of a polymorphism (see Examples herein).

According to another aspect of the present invention there is provided a human KDR gene or its complementary strand comprising a variant allelic polymorphism at one or more of the positions defined herein or a fragment thereof of at least 20 bases comprising at least one novel polymorphism.

Fragments are at least 20 bases, more preferably at least 25 bases, more preferably at least 30 bases.

According to another aspect of the present invention there is provided a polynucleotide comprising at least 20 bases of the human KDR gene and comprising an allelic variant selected from any one of the following:

| **Region** | **Variant** | **SEQ ID NO:** |
|---|---|---|
| Promoter | 162 C | |
| | 423 G | |
| | 461 C | SEQ ID NO: 1 |

| **Region** | **Variant** | **SEQ ID NO:** |
|---|---|---|
| exon 1 | 26 A | SEQ ID NO: 24 |
| exon 7 | 224 A | SEQ ID NO: 2 |
| exon 11 | 145 T | SEQ ID NO: 6 |

| **Region** | **Variant** | **SEQ ID NO:** |
|---|---|---|
| intron adjacent exon 2 | 345 A | SEQ ID NO: 2 |
| intron adjacent exon 6 | 112 A | |
| | 329 A | SEQ ID NO: 3 |
| intron adj. exon 9 | 339 A | SEQ ID NO: 5 |
| intron adj. exon 11 | 103 C | SEQ ID NO: 6 |
| intron adjacent exon 21 | 32 G | |
| | 94 T | SEQ ID NO: 7 |

According to another aspect of the present invention there is provided a nucleic acid comprising a nucleic acid sequence of at least 20 bases possessing any one of the following polymorphisms:
162C, 423G, 461C (each according to SEQ ID NO: 1); 345A (according to SEQ ID NO: 2); 112A, 329A (each according to SEQ ID NO: 3); 224A (according to SEQ ID NO: 4); 339A (according to SEQ ID NO: 5); 103C, 145T (each according to SEQ ID NO: 6); 32G and 94T (each according to SEQ ID NO: 7),
or a complementary strand thereof comprising at least one of the said polymorphisms.

According to another aspect of the present invention there is provided an isolated or recombinantly produced nucleic acid comprising a nucleic acid sequence of at least 20 bases possessing any one of the following polymorphisms: 162C, 423G, 461C (each according to SEQ ID NO: 1); 345A (according to SEQ ID NO: 2); 112A, 329A (each according to SEQ ID NO: 3); 224A (according to SEQ ID NO: 4); 339A (according to SEQ ID NO: 5); 103C, 145T (each according to SEQ ID NO: 6); 32G, 94T (each according to SEQ ID NO: 7); and 26A (according to SEQ ID NO: 24),
or a complementary strand thereof comprising at least one of the said polymorphisms.

According to another aspect of the present invention there is provided a human KDR gene or its complementary strand comprising a polymorphism, preferably corresponding with one or more of the positions defined herein, or a fragment thereof of at least 20 bases comprising at least one polymorphism.

The invention further provides nucleotide primers which detect the KDR gene polymorphisms of the invention. Such primers can be of any length, for example between 8 and 100 nucleotides in length, but will preferably be between 12 and 50 nucleotides in length, more preferable between 17 and 30 nucleotides in length.

According to another aspect of the present invention there is provided an allele specific primer capable of detecting a KDR gene polymorphism, preferably at one or more of the positions as defined herein.

An allele specific primer is used, generally together with a constant primer, in an amplification reaction such as a PCR reaction, which provides the discrimination between alleles through selective amplification of one allele at a particular sequence position e.g. as used for ARMS™-allele specific amplification assays. The allele specific primer is preferably 17- 50 nucleotides, more preferably about 17-35 nucleotides, more preferably about 17-30 nucleotides.

An allele specific primer preferably corresponds exactly with the allele to be detected but derivatives thereof are also contemplated wherein about 6-8 of the nucleotides at the 3' terminus correspond with the allele to be detected and wherein up to 10, such as up to 8, 6, 4, 2, or 1 of the remaining nucleotides may be varied without significantly affecting the properties of the primer. Often the nucleotide at the -2 and/or -3 position (relative to the 3' terminus) is mismatched in order to optimise differential primer binding and preferential extension from the correct allele discriminatory primer only.

Primers may be manufactured using any convenient method of synthesis. Examples of such methods may be found in standard textbooks, for example "Protocols for Oligonucleotides and Analogues; Synthesis and Properties," Methods in Molecular Biology Series; Volume 20; Ed. Sudhir Agrawal, Humana ISBN: 0-89603-247-7; 1993; 1^{st} Edition. If required the primer(s) may be labelled to facilitate detection.

According to another aspect of the present invention there is provided an allele-specific oligonucleotide probe capable of detecting a KDR gene polymorphism, preferably at one or more of the positions defined herein.

The allele-specific oligonucleotide probe is preferably 17-50 nucleotides, more preferably about 17-35 nucleotides, more preferably about 17-30 nucleotides.

The design of such probes will be apparent to the molecular biologist of ordinary skill. Such probes are of any convenient length such as up to 50 bases, up to 40 bases, more conveniently up to 30 bases in length, such as for example 8-25 or 8-15 bases in length. In general such probes will comprise base sequences entirely complementary to the corresponding wild type or variant locus in the gene. However, if required one or more mismatches may be introduced, provided that the discriminatory power of the oligonucleotide probe is not unduly affected. The probes of the invention may carry one or more labels to facilitate detection, such as in Molecular Beacons. Single stranded oligonucleotides corresponding to SEQ ID NOs: 8, 9 or their complement, could be used as probes to detect the particular polymorphism at the central position (emboldened). The probe would bind more efficiently to a target sequence that possessed the particular complementary polymorphism base at this central (polymorphism) location than one with a base mismatch.

According to another aspect of the present invention there is provided an allele specific primer or an allele specific oligonucleotide probe capable of detecting a KDR gene polymorphism at one of the positions defined herein.

According to another aspect of the present invention there is provided a diagnostic kit comprising an allele specific oligonucleotide probe of the invention and/or an allele-specific primer of the invention.

The diagnostic kits may comprise appropriate packaging and instructions for use in the methods of the invention. Such kits may further comprise appropriate buffer(s) and polymerase(s) such as thermostable polymerases, for example taq polymerase. Such kits may also comprise companion primers and/or control primers or probes. A companion primer is one that is part of the pair of primers used to perform PCR. Such primer usually complements the template strand precisely.

In another aspect of the invention, the polymorphisms of this invention may be used as genetic markers in linkage studies. This particularly applies to the polymorphisms of relatively high frequency. The KDR gene has been localised to chromosome 4q11-q12 (Sait et al (**1995**) Cytogen Cell Genet 70,145-146; Spritz et al (**1994**) Genomics 22,431-436). Low frequency polymorphisms may be particularly useful for haplotyping as described below. A haplotype is a set of alleles found at linked polymorphic sites (such as within a gene) on a single (paternal or maternal) chromosome. If recombination within the gene is random, there may be as many as 2ⁿ haplotypes, where 2 is the number of alleles at each SNP and n is the number of SNPs. One approach to identifying mutations or polymorphisms which are correlated with clinical response is to carry out an association study using all the haplotypes that can be identified in the population of interest. The frequency of each haplotype is limited by the frequency of its rarest allele, so that SNPs with low frequency alleles are particularly useful as markers of low frequency haplotypes. As particular mutations or polymorphisms associated with certain clinical features, such as adverse or abnormal events, are likely to be of low frequency within the population, low frequency SNPs may be particularly useful in identifying these mutations (for examples see: Linkage disequilibrium at the cystathionine beta synthase (CBS) locus and the association between genetic variation at the CBS locus and plasma levels of homocysteine. *Ann Hum Genet* (1998) **62**:481-90, De Stefano V, Dekou V, Nicaud V, Chasse JF, London J, Stansbie D, Humphries SE, and Gudnason V; and Variation at the von willebrand factor (vWF) gene locus is associated with plasma vWF:Ag levels: identification of three novel single nucleotide polymorphisms in the vWF gene promoter. *Blood* (1999) **93**:4277-83, Keightley AM, Lam YM, Brady JN, Cameron CL, Lillicrap D).

The nucleic acid sequences of the invention, particularly those relating to and identifying the single nucleotide polymorphisms identified herein represent a valuable information source with which to characterise individuals in terms of, for example, their identity, haplotype and other sub-groupings, such as susceptibility to treatment with particular drugs. These approaches are most easily facilitated by storing the sequence information in a computer readable medium and then using the information in standard macromolecular structure programs or to search sequence databases using state of the art searching tools such as GCG (Genetics Computer Group), BlastX BlastP, BlastN, FASTA (refer to Altschul et al. J. Mol. Biol. **215**:403-410, 1990). Thus, the polymorphism containing nucleic acid sequences of the invention are particularly useful as components in databases useful for sequence identity, genome mapping, pharmacogenetics and other search analyses. Generally, the sequence information relating to the nucleic acid sequences and polymorphisms of the invention may be reduced to, converted into or stored in a tangible medium, such as a computer disk, preferably in a computer readable form. For example, chromatographic scan data or peak data, photographic scan or peak data, mass spectrographic data, sequence gel (or other) data.

According to another aspect of the present invention there is provided a computer readable medium comprising at least one novel sequence of the invention stored on the medium. The computer readable medium may be used, for example, in homology searching, mapping, haplotyping, genotyping or pharmacogenetic analysis. The computer readable medium can be any composition of matter used to store information or data, including, for example, floppy disks, tapes, chips, compact disks, digital disks, video disks, punch cards and hard drives.

According to another aspect of the present invention there is provided a method of treating a human in need of treatment with a small molecule drug acting on the KDR (VEGFR2) protein or an anti-sense oligonucleotide or ribozyme acting against the KDR (VEGFR2) mRNA, in which the method comprises:
i) diagnosis of a polymorphism in the KDR gene in the human, which diagnosis preferably comprises determining the sequence at one or more of the following positions: positions 162, 423 and 461 in the promoter region of the KDR gene (as defined by the position in SEQ ID NO: 1); position 224 (as defined by the position in SEQ ID NO: 4), and 145 (as defined by the position in SEQ ID NO: 6) in the coding region of the KDR gene; positions 345 (as defined by the position in SEQ ID NO: 2), 112, 329 (as defined by the position in SEQ ID NO: 3), 339 (as defined by the position in SEQ ID NO: 5), 103 (as defined by the position in SEQ ID NO: 6), 32 and 94 (as defined by the position in SEQ ID NO: 7) in the intron region of the KDR gene; and, amino acid residues 297, and 472 in the KDR polypeptide as defined by the position in EMBL Accession No: AF035121;
ii) determining the status of the human by reference to polymorphism in the KDR gene; and,
iii) administering an effective amount of the drug.

According to another aspect of the present invention there is provided a method of treating a human in need of treatment with a small molecule drug acting on the KDR (VEGFR2) protein or an anti-sense oligonucleotide or ribozyme acting against the KDR (VEGFR2) mRNA, in which the method comprises:
i) diagnosis of a polymorphism in the KDR gene in the human, which diagnosis preferably comprises determining the sequence at one or more of the following positions: positions 162, 423 and 461 in the promoter region of the KDR gene (as defined by the position in SEQ ID NO: 1); position 224 (as defined by the position in SEQ ID NO: 4), and 145 (as defined by the position in SEQ ID NO: 6) in the coding region of the KDR gene; positions 345 (as defined by the position in SEQ ID NO: 2), 112, 329 (as defined by the position in SEQ ID NO: 3), 339 (as defined by the position in SEQ ID NO: 5), 103 (as defined by the position in SEQ ID NO: 6); 32, 94 (as defined by the position in SEQ ID NO: 7) in the intron region of the KDR gene; 26 in the 5' UTR (as defined by the position in SEQ ID NO: 24) in the exon 1 region of the KDR gene; and, amino acid residues 297, and 472 in the KDR polypeptide as defined by the position in EMBL Accession No: AF035121;
ii) determining the status of the human by reference to polymorphism in the KDR gene.; and
iii) administering an effective amount of the drug.

Preferably determination of the status of the human is clinically useful. Examples of clinical usefulness include deciding which drug or drugs to administer and/or in deciding on the effective amount of the drug or drugs. The term "drug acting at KDR" means that drug binding with the KDR protein in humans is an important part of a drug exerting its pharmaceutical effect in man.

According to another aspect of the present invention there is provided use of a drug acting on the KDR (VEGFR2) protein or the KDR (VEGFR2) mRNA in preparation of a medicament for treating a disease in a human diagnosed as having a polymorphism therein, preferably at one or more of the positions defined herein.

According to another aspect of the present invention there is provided a pharmaceutical pack comprising a drug acting as a KDR antagonist and instructions for administration of the drug to humans diagnostically tested for a polymorphism therein, preferably at one or more of the positions defined herein.

According to another aspect of the present invention there is provided an allelic variant of human KDR polypeptide comprising at least one of the following:
a isoleucine at position 297, and a histidine at position 472 of the polypeptide disclosed in EMBL Accession No. AF035121, or a fragment thereof comprising at least 10 amino acids provided that the fragment comprises at least one allelic variant.

Fragments of a polypeptide are at least 10 amino acids, more preferably at least 15 amino acids, more preferably at least 20 amino acids.

According to another aspect of the present invention there is provided an antibody specific for an allelic variant of human KDR polypeptide as described herein.

Antibodies can be prepared using any suitable method. For example, purified polypeptide may be utilized to prepare specific antibodies. The term "antibodies" is meant to include polycional antibodies, monoclonal antibodies, and the various types of antibody constructs such as for example F(ab')₂, Fab and single chain Fv. Antibodies are defined to be specifically binding if they bind the allelic variant of KDR with a Kₐ of greater than or equal to about 10⁷ M⁻¹. Affinity of binding can be determined using conventional techniques, for example those described by Scatchard et al., *Ann. N.Y. Acad. Sci.,* (1949) **51**:660.

Polyclonal antibodies can be readily generated from a variety of sources, for example, horses, cows, goats, sheep, dogs, chickens, rabbits, mice or rats, using procedures that are well-known in the art. In general, antigen is administered to the host animal typically through parenteral injection. The immunogenicity of antigen may be enhanced through the use of an adjuvant, for example, Freund's complete or incomplete adjuvant. Following booster immunizations, small samples of serum are collected and tested for reactivity to antigen. Examples of various assays useful for such determination include those described in: *Antibodies: A Laboratory Manual,* Harlow and Lane (eds.), Cold Spring Harbor Laboratory Press, 1988; as well as procedures such as countercurrent immuno-electrophoresis (CIEP), radioimmunoassay, radioimmunoprecipitation, enzyme-linked immuno-sorbent assays (ELISA), dot blot assays, and sandwich assays, see U.S. Patent Nos. 4,376,110 and 4,486,530.

Monoclonal antibodies may be readily prepared using well-known procedures, see for example, the procedures described in U.S. Patent Nos. RE 32,011; 4,902,614; 4,543,439 and 4,411,993; Monoclonal Antibodies, Hybridomas: *A New Dimension in Biological Analyses,* Plenum Press, Kennett, McKearn, and Bechtol (eds.), (1980).

The monoclonal antibodies of the invention can be produced using alternative techniques, such as those described by Alting-Mees et al., "Monoclonal Antibody Expression Libraries: A Rapid Alternative to Hybridomas", *Strategies in Molecular Biology* (1990) **3**:1-9, which is incorporated herein by reference. Similarly, binding partners can be constructed using recombinant DNA techniques to incorporate the variable regions of a gene that encodes a specific binding antibody. Such a technique is described in Larrick et al., *Biotechnology,* (1989) **7**: 394.

Once isolated and purified, the antibodies may be used to detect the presence of antigen in a sample using established assay protocols, see for example "A Practical Guide to ELISA" by D. M. Kemeny, Pergamon Press, Oxford, England.

According to another aspect of the invention there is provided a diagnostic kit comprising an antibody of the invention.

The invention will now be illustrated but not limited by reference to the following Examples. All temperatures are in degrees Celsius.

In the Examples below, unless otherwise stated, the following methodology and materials have been applied.

AMPLITAQ™ ,available from Perkin-Elmer Cetus, is used as the source of thermostable DNA polymerase.

General molecular biology procedures can be followed from any of the methods described in "Molecular Cloning - A Laboratory Manual" Second Edition, Sambrook, Fritsch and Maniatis (Cold Spring Harbor Laboratory, 1989) or "Current Protocols in Molecular Biology Volumes1-3 ,Edited by FM Asubel, R Brent, RE Kingston pub John Wiley 1998

Electropherograms were obtained in a standard manner: data was collected by ABI377 data collection software and the wave form generated by ABI Prism sequencing analysis (2.1.2).

### EXAMPLE 1

### Identification of Polymorphisms

### 1. Methods

### DNA Preparation

DNA was prepared from frozen blood samples collected in EDTA following protocol I (Molecular Cloning: A Laboratory Manual, p392, Sambrook, Fritsch and Maniatis, 2^{nd} Edition, Cold Spring Harbor Press, 1989) with the following modifications. The thawed blood was diluted in an equal volume of standard saline citrate instead of phosphate buffered saline to remove lysed red blood cells. Samples were extracted with phenol, then phenol/chloroform and then chloroform rather than with three phenol extractions. The DNA was dissolved in deionised water. 29 individual samples were analysed

### Template Preparation

Templates were prepared by PCR using the oligonucleotide primers and annealing temperatures set out below. The extension temperature was 72° and denaturation temperature 94°. Generally 50 ng of genomic DNA was used in each reaction and subjected to 35 cycles of PCR. Where described below, the primary fragment was diluted 1/100 and two microlitres were used as template for amplification of secondary fragments. PCR was performed in two stages (primary fragment then secondary fragment) to ensure specific amplification of the desired target sequence.

### Novel polymorphisms within coding region of KDR

### (1) G/A polymorphism at position 224 of SEQ ID NO: 4

| **Position** | **Polymorphism** | **Allele Frequency** |
|---|---|---|
| 224 | G/A | G 89% A 11% |

Polymorphism at position 224 results in a codon change from Valine (GTA) to Isoleucine (ATA) at codon position 297 (codon numbering according to EMBL Accession Number AF035121). Codon 297 is located within exon 7 and amino acid residue 297 forms part of the third Ig like domain in the extracellular region of KDR (Terman et al., (1992) Biochem Biophys Res Comm **187**:1579-1586.). The third Ig like domain is thought to play a role in ligand binding (Lu et al., *supra).*

The sequences flanking the G/A polymorphism at position 224 are given below (positions 174 - 274, numbering according to SEQ ID No: 4) 
AGTCTGGGAGTGAGATGAAGAAATTTTTGAGCACCTTAACTATAGATGGTRTAAC
CCGGAGTGACCAAGGATTGTACACCTGTGCAGCATCCAGTGGGCTG (SEQ ID
NO:10)
 wherein R = G or A

Engineering of nucleotide 225 (T-C) creates a polymorphic RsaI site (GTAC) which can be used as a diagnostic assay for the polymorphism at position 224. Forward primer 1-20
Engineered primer 225-244 5' TCCTTGGTCACTCCGGGTTG (SEQ ID NO: 25) Altered base in bold

A PCR product (244bp) generated from a wild type individual (G variant) will not be cleaved on digestion with RsaI (New England Biolabs). Digestion of a product derived from a heterozygote individual will generate products of 244 bp, 224bp and 20 bp. Digestion of a product derived from an individual homozygous for the A variant will give rise to products of 224bp and 20 bp.

### (2) A/T polymorphism at position 145 of SEQ ID NO: 6

| **Position** | **Polymorphism** | **Allele Frequency** |
|---|---|---|
| 145 | A/T | A 73% T 27% |

Polymorphism at position 145 results in a codon change from Glutamine (CAA) to histidine (CAT) at codon position 472 (codon numbering according to EMBL Accession Number AF035121). Codon 472 is located within exon 11 and amino acid residue 472 is located in the fifth Ig like domain in the extracellular region of KDR (Terman et al., 1992, *supra*).

The sequences flanking the A/T polymorphism (W) at position 145 are given below (positions 95-195, numbering according to SEQ ID No: 6). 
TAAAGAAAAATTAAATCATAATATGCGCTGTTATCTCTTCTTATAGCCAWGCTG
TCTCAGTGACAAACCCATACCCTTGTGAAGAATGGAGAAGTGTGGAGGA (SEQ ID
NO:11)
 wherein W = A or T

Polymorphism at position 145 (A/T) creates an NlaIII restriction site (CATG) which can be used as a diagnostic test. A PCR product (499 bp) generated from a wild type individual (A variant) will not be cleaved on digestion with NlaIII (New England Biolabs). Digestion of a product derived from a heterozygote individual will generate products of 146 bp, 353 bp and 499 bp. Digestion of a PCR product derived from an individual homozygous for the T variant will generate products of 146 bp and 353 bp.

### Novel polymorphisms within the Promoter of the KDR gene (SEQ ID NO: 1, positions 16-534 EMBL Accession Number No X89776)

### (1) T/C polymorphism at position 162 (SEQ ID No: 1)

| **Position** | **Polymorphism** | **Allele Frequency** |
|---|---|---|
| T/C | 162 | T 75% C 25% |

The sequences flanking the T/C polymorphism (Y) at position 162 are given below (positions 112-212 of SEQ ID NO: 1) 
AGGTCACTTCAAACTTGGAGCCGCCAAATATTTGGGAAATAGCGGAATGYTGG
CGAACTGGGCAAGTGCGTTTCTGATTAAGAGCAACCAGATTCAGCT (SEQ ID
NO:12)
 wherein Y = T or C

### (2) C/G polymorphism at position 423 (SEQ ID NO:1)

| Position | Polymorphism | Allele Frequency |
|---|---|---|
| C/G | 423 | C 78% G 22% |

The sequences flanking the C/G polymorphism (S) at position 423 are given below (corresponds to positions 373-473 of SEQ ID NO: 1) 
GCGCGATGGCGAAGAGGGTCCTGCACTTTGACGCGCCTGGTGAGGGAGCGSTGCT
CTTCGCAGCGCTCCTGGTGATGCTCCCCAAATTTCGGGGACCGGC (SEQ ID NO:
13)
 wherein S = C or G

### (3) T/C polymorphism at position 461 (SEQ ID NO: 1)

| **Position** | **Polymorphism** | **Allele Frequency** |
|---|---|---|
| T/C | 461 | T 79% C 21% |

The sequences flanking the T/C polymorphism (Y) at position 461 are given below (corresponds to positions 411-511 of SEQ ID NO: 1) 
GGTGAGGGAGCGGTGCTCTTCGCAGCGCTCCTGGTGATGCTCCCCAAATTYCGGG
GACCGGCAAGCGATTAAATCTTGGAGTTGCTCAGCGCCCGTTACCG (SEQ ID
NO:14)
 wherein Y = T or C

### Novel polymorphisms in flanking intronic sequence

### (1) G/A polymorphism at position 345 (Exon 2, SEQ ID NO: 2)

| **Position** | **Polymorphism** | **Allele Frequency** |
|---|---|---|
| 345 | G/A | G 95% A 5% |

The sequences flanking the G/A polymorphism (**R**) at position 345 (of SEQ ID NO:2) are shown below (corresponds to positions 295-395 of SEQ ID NO: 2) 
GATTTCTTGTGTTAAGTAACTGATTGTTTATTGAGTGGAAATAATTTCCARTAGAG
CAGAATTATAATAGAGCTTGTAGTAATTGTTCATAAGTGGTGAGG (SEQ ID NO:15)
 wherein R = G or A

### (2) G/A polymorphism at position 112 (Exon 6, SEQ ID NO: 3)

| **Position** | **Polymorphism** | **Allele Frequency** |
|---|---|---|
| 112 | G/A | G 74% A 26% |

The sequences flanking the G/A polymorphism (**R**) at position 112 (according to SEQ ID NO:3) are shown below (= positions 62-162 of SEQ ID NO: 3) 
AAAAAATAAATCTGTGCAAAGTTATAGGCTTATTTGCTCTCTCATGTTCTRTTTTT
TCAATTTACTTGCTCTAGGGTATAGGATTTATGATGTGGTTCTGA (SEQ ID NO:16)
 wherein R = G or A

### (3) G/A polymorphism at position 329 (Exon 6, SEQ ID NO: 3)

| **Position** | **Polymorphism** | **Allele Frequency** |
|---|---|---|
| 329 | G/A | G 59% A 41% |

The sequences flanking the G/A polymorphism (**R**) at position 329 (according to SEQ ID NO:3) are shown below (= positions 279-379 of SEQ ID NO: 3) 
ACGCTAATGATTCAAAGCCAGACCTCCAAATACTTACATAATAAGCCCCARTGAA
GTTTGCTTGAGAGATAGGGGCCTCTTTGGCCAGATAAAATGTAAGA (SEQ ID
NO:17)
 wherein R = G or A

### (4) G/A polymorphism at position 339 (Exon 9, SEQ ID NO:5)

| **Position** | **Polymorphism** | **Allele Frequency** |
|---|---|---|
| 339 | G/A | G 74% A 26% |

The sequences flanking the G/A polymorphism (**R**) at position 339 (according to SEQ ID NO:6) are shown below (= 289-377 of SEQ ID NO: 6) 
ACCACCGTTCCTCTGCCTCCTGCTGCTTCACTCATATCATGGCTGGGCCTRCGTAC
AAAAGTCATCTGGCGTGGTGAAGCTGAAGTGAA (SEQ ID NO:18)
 wherein R = G or A

### (5) A/C polymorphism at position 103 (Exon 11, SEQ ID NO: 6)

| **Position** | **Polymorphism** | **Allele Frequency** |
|---|---|---|
| 103 | A/C | A 98% C 2% |

The sequences flanking the A/C polymorphism (**M**) at position 103 (according to SEQ ID NO:7) are shown below (= positions 53-153 of SEQ ID NO: 6) 
AATAACCAAAGTCTGAATCTTTTCCTTACTCTTGACTCTAATTAAAGAAAMATTA
AATCATAATATGCGCTGTTATCTCTTTCTTATAGCCAAGCTGTCTC (SEQ ID NO:19)
 wherein M = A or C

### (6) T/G polymorphism at position 32 (Exon 21, SEQ ID NO: 7)

| **Position** | **Polymorphism** | **Allele Frequency** |
|---|---|---|
| 32 | T/G | T 98% G 2% |

The sequences flanking the T/G polymorphism (**K**) at position 32 (according to SEQ ID NO:7) are shown below (=positions1-82 of SEQ ID NO:7) 
TTGATGTCCTCCTTGTCTGCTTTTAGTAGTKTCAATTATCTCCATGGTTTACTACA
TTTTAAAGGTTGTAAACTTTTAAAG (SEQ ID NO:20)
 wherein K = T or G

### (7) A/T polymorphism at position 94 (Exon 21, SEQ ID NO: 7)

| **Position** | **Polymorphism** | **Allele Frequency** |
|---|---|---|
| 94 | A/T | A 68% T 32% |

The sequences flanking the A/T polymorphism (**W**) at position 94 (according to SEQ ID NO:8) are shown below (= positions 44-144 of SEQ ID NO: 7) 
CATGGTTTACTACATTTTAAAGGTTGTAAACTTTTAAAGACTCATTTTGTWTTCAA
GGAGTTTGTTTGTTCCTTTGCTTTTTATAGACCAAAGGGGCACG (SEQ ID NO:21)
 wherein W = A or T

### Novel polymorphism in 5' UTR of the KDR gene

### (1) G/A polymorphism at position 26 (exon 1, SEQ ID NO: 24)

| **Position** | **Polymorphism** | **Allele Frequency** |
|---|---|---|
| 26 | G/A | G 70% A 30% |

The sequences flanking the G/A polymorphism (**R**) at position 26 (according to SEQ ID NO:24) are shown below (= positions 193-243 of SEQ ID NO: 22): TCCCGGGACCCCGGGAGAGCGGTCARTGTGTGGTCGCTGCGTTTCCTCTGC wherein R = G or A
SEQ ID NO: 24 is a sub-sequence of SEQ ID NO: 23, which is a sub-sequence of SEQ ID NO: 22.

### EXAMPLE 2

### Haplotype analysis of KDR polymorphisms

Two coding polymorphisms (Valine 297 Isoleucine; Glutamine 472 Histidine) in the KDR gene were used in conjunction with an intronic SNP (G/A Position 339 according to SEQ ID NO: 5) to predict haplotype frequencies.

| Polymorphism | Allele frequency |
|---|---|
| Codon 297 Val (GTA)/Ile (ATA) | G 89% A 11% |
| Intron Position 339 in SEQ ID NO: 5 | G 74% A 26% |
| Codon 472 Gln (CAA)/His (CAT) | A 73% T 27% |

The polymorphisms span a distance of 6.5 kb, genotypes in 73 individuals were determined by sequencing and haplotype frequencies were determined using a combination of Clark's algorithm and the EH (end haplotype) software. Only 5 haplotypes were observed in the 73 individuals.

| Haplotype | Frequency (%) |
|---|---|
| GGA | 40 |
| GGT | 24 |
| GAA | 26 |
| AGT | 6 |
| AAA | 4 |

### Sequence listing notes:

### SEQ ID NO: 1 (Corresponds to positions 16-534 of EMBL Accession No X89776) Positions 1-519 ( Promoter region)

| | | | |
|---|---|---|---|
| PolymorphismT/C | 162 | | |
| C/G | 423 | | |
| T/C | 461 | | |
| Forward primer | 1-20; | Reverse primer | 500-519 |

### SEQ ID NO: 2 (Corresponds to positions 150523-151026 of EMBL Accession AC 021220)

| | | |
|---|---|---|
| Flanking intron | 1-178 | |
| Exon 2 | 178-272 | |
| Flanking intron | 273-503 | |
| Polymorphism G/A | 345 | |
| Forward primer | 1-20; Reverse primer | 484-503 |

### SEQ ID NO: 3 (Corresponds to positions 143659-144055 of EMBL Accession AC 021220)

| | | |
|---|---|---|
| Forward primer | 1-20; Reverse primer | 388-397 |
| Flanking intron | 1-135 | |
| Exon 6 | 136-275 | |
| Flanking intron | 276-397 | |
| Polymorphism G/A | 112 | |
| Polymorphism G/A | 329 | |

### SEO ID NO: 4

| | | |
|---|---|---|
| Forward primer | 1-20; Reverse primer | 434-453 |
| Flanking intron | 1-133 | |
| Exon 7 | 134-311 | |
| Flanking intron | 312-453 | |
| Polymorphism G/A | 224 | |

Position 1-393 corresponds to positions 143269-142877 of AC021220. Positions 394-453 correspond to novel sequence which was determined by sequencing of BAC clone 81A1.

### SEQ ID NO: 5 (Corresponds to 11289-113265 of EMBL Accession Number AC021220)

| | | |
|---|---|---|
| Forward primer | 1-20; Reverse primer | 358-377 |
| Flanking intron | 1-57 | |
| Exon 9 | 58-221 | |
| Flanking intron | 221-377 | |
| Polymorphism G/A | 339 | |

### SEQ ID NO: 6 (Corresponds to 109095-109593 of EMBL Accession Number AC021220)

| | | |
|---|---|---|
| Forward primer | 1-20; Reverse primer | 480-499 |
| Flanking intron | 1-141 | |
| Exon 11 | 142-275 | |
| Flanking intron | 276-499 | |
| Polymorphism A/C | 103 | |
| Polymorphism A/T | 145 | |

### SEQ ID NO: 7 (Corresponds to 79362-79718 of AC021220)

| | | |
|---|---|---|
| Forward primer | 1-20; Reverse primer | 338-357 |
| Flanking intron | 1-130 | |
| Exon 21 | 131-284 | |
| Flanking intron | 285-357 | |
| Polymorphism T/G | 32 | |
| Polymorphism A/T | 94 | |

### SEQ ID NO: 22 (Intron sequence derived from EMBL Accession AC021220 Positions 187 - 556 correspond to positions 1 - 370 EMBL Accession AF035121 Position 218 corresponds to position 32 in EMBL Accession AF035121)

| | | |
|---|---|---|
| Flanking Intron | 1-186 | |
| Exon 1 | 187-556 | |
| Flanking Intron | 557-628 | |
| Polymorphism G/A | 218 | |
| Forward primer | 1 - 24; Reverse primer | 605 - 628 |

### SEQ ID NO: 23 (Intron sequence derived from EMBL Accession AC021220 Positions 124-315 correspond to positions 1 - 192 EMBL Accession AF035121 Position 145 corresponds to position 32 in EMBL Accession AF035121)

| | | |
|---|---|---|
| Flanking Intron | 1-123 | |
| Exon 1 | 124-315 | |
| Polymorphism | G/A 145 | |
| Forward primer | 1-24; Reverse primer | 292-315 |

## Claims

1. A method for the diagnosis or detection of a polymorphism in KDR in a human, which method comprises determining the sequence of the human at at least one polymorphic position of KDR and determining the status of the human by reference to polymorphism in KDR.

2. A method according to claim 1, wherein the polymorphic position is selected from the group consisting of position:
224 according to SEQ ID NO: 4;
145, 103 according to SEQ ID NO:6;
162, 423, 461 according to SEQ ID NO: 1;
345 according to SEQ ID NO: 2;
112, 329 according to SEQ ID NO: 3;
339 according to SEQ ID NO:5;
32, 94 according to SEQ ID NO:7; and,
26 according to SEQ ID NO:24.

3. A method for the diagnosis of a polymorphism in KDR in a human, which method comprises determining the sequence of the human at one or more positions selected from the group consisting of: position 224 (as defined by the position in SEQ ID NO: 4), and 145 (as defined by the position in SEQ ID NO:6) in the coding region of the KDR gene; positions 162, 423 and 461 in the promoter region of the KDR gene (as defined by the position in SEQ ID NO: 1); positions 345 (as defined by the position in SEQ ID NO: 2), 112 and 329 (as defined by the position in SEQ ID NO: 3), 339 (as defined by the position in SEQ ID NO:5), 103 (as defined by the position in SEQ ID NO:6); 32, 94 (as defined by the position in SEQ ID NO:7) in the intron region of the KDR gene; position 26 in the 5' UTR region of the KDR gene (as defined by the position in SEQ ID NO: 24); and, codons 297 and 472 in the KDR polypeptide as defined by the position in EMBL Accession No: AF035121, and determining the status of the human by reference to polymorphism in KDR.

4. A method according to claim 2 or claim 3, in which the single nucleotide polymorphism at position 224 (according to the position in SEQ ID NO: 4) is the presence of G and/or A; at position 145 (according to the position in SEQ ID NO:6) is the presence of A and/or T; at position 162 (according to the position in SEQ ID NO: 1) is the presence of T and/or A; at position 423 (according to the position in SEQ ID NO: 1) is the presence of C and/or G; at position 461(according to the position in SEQ ID NO: 1) is the presence of T and/or C; at position 345 (according to the position in SEQ ID NO: 2) is the presence of G and/or A; at position 112 (according to the position in SEQ ID NO: 3) is the presence of G and/or A; at position 329 (according to the position in SEQ ID NO: 3) is the presence of G and/or A; at position 339 (according to the position in SEQ ID NO:5) is the presence of G and/or A; at position 103 (according to the position in SEQ ID NO:6) is the presence of A and/or C; at position 32 (according to the position in SEQ ID NO: 7) is the presence of T and/or G; at position 94 (according to the position in SEQ ID NO:7) is the presence of A and/or T; and, at position 26 (according to the position in SEQ ID NO: 24) is the presence of G and/or A.

5. A nucleic acid polymorphism detecting method as claimed in any of claims 2 - 4, wherein the nucleic acid region containing the potential single nucleotide polymorphism is amplified by polymerase chain reaction prior to determining the sequence.

6. A nucleic acid polymorphism detecting method as claimed in any of claims 2 - 5, wherein the presence or absence of the single nucleotide polymorphism is detected by reference to the loss or gain of, optionally engineered, sites recognised by restriction enzymes.

7. A nucleic acid polymorphism detecting method as claimed in any of claims 2 - 5, wherein the particular polymorphism is determined by a method selected from ARMS-allele specific amplification, allele specific hybridisation, oligonucleotide ligation assay or restriction fragment length polymorphism (RFLP).

8. An amino acid polymorphism detecting method as claimed in any of claims 2 - 5, wherein the presence of a polymorphic amino acid residue in the KDR protein is determined by immunological methods such as enzyme linked immunosorbent assay (ELISA).

9. A method as claimed in any of the preceding claims for use in assessing the predisposition and/or susceptibility of an individual to diseases mediated by VEGF.

10. A method for the diagnosis or prognosis of VEGF-mediated disease, which method comprises:
i) obtaining sample nucleic acid from an individual;
ii) detecting the presence or absence of a variant nucleotide at one or more of positions: 224 (according to SEQ ID NO: 4): 145, 103 (each according to SEQ ID NO: 6); 162, 423, 461 (each according to SEQ ID NO: 1); 345 (according to SEQ ID NO: 2); 112, 329 (each according to SEQ ID NO: 3); 339 (according to SEQ ID NO: 5); 32, 94 (each according to SEQ ID NO: 7); and, 26 (according to SEQ ID NO:24), in the KDR gene; and,
iii) determining the diagnostic or prognostic status of the individual by reference to polymorphism in the KDR gene.

11. A method for the diagnosis of VEGF-mediated disease, which method comprises:
i) obtaining a protein containing sample from an individual;,
ii) detecting the presence or absence of a polymorphic amino acid at either or both amino acid positions 297 and 472 (according to the position in EMBL Accession No. AF035121); and,
iii) determining the status of the human by reference to the presence or absence of a polymorphism in KDR.

12. A method as claimed in claim 11, wherein the polymorphism is either or both of: Val297Ile or Gln472His.

13. An isolated polynucleotide comprising at least 20 bases of the human KDR gene and comprising an allelic variant selected from any one of the following:
| **Region** | **Variant** | **SEQ ID NO:** |
|---|---|---|
| Promoter | 162 C | |
| | 423 G | |
| | 461 C | SEQ ID NO: 1 |
| **Region** | **Variant** | **SEQ ID NO:** |
|---|---|---|
| exon 1 | 26 A | SEQ ID NO: 24 |
| exon 7 | 224 A | SEQ ID NO: 2 |
| exon 11 | 145 T | SEQ ID NO: 6 |
| **Region** | **Variant** | **SEQ ID NO:** |
|---|---|---|
| intron adjacent exon 2 | 345 A | SEQ ID NO: 2 |
| intron adjacent exon 6 | 112 A | |
| | 329 A | SEQ ID NO: 3 |
| intron adj. exon 9 | 339 A | SEQ ID NO: 5 |
| intron adj. exon 11 | 103 C | SEQ ID NO: 6 |
| intron adjacent exon 21 | 32 G | |
| | 94 T | SEQ ID NO: 7 |

14. An isolated nucleic acid comprising a nucleic acid sequence of at least 20 bases possessing any one of the following polymorphisms: 224A (according to SEQ ID NO: 4); 145T, 103C (each according to SEQ ID NO: 6); 162C, 423G, 461C (each according to SEQ ID NO: 1); 345A (according to SEQ ID NO: 2); 112A, 329A (each according to SEQ ID NO: 3); 339A (according to SEQ ID NO: 5); 32G, 94T (each according to SEQ ID NO: 7); or 26A (according to SEQ ID NO: 24), or a complementary strand thereof comprising at least one of the said polymorphisms.

15. A diagnostic nucleic acid primer capable of detecting a polymorphism in the KDR gene at one or more of positions: 224 (according to SEQ ID NO: 4); 145, 103 (each according to SEQ ID NO: 6); 162, 423, 461 (each according to SEQ ID NO: 1); 345 (according to SEQ ID NO: 2); 112, 329 (each according to SEQ ID NO: 3); 339 (according to SEQ ID NO: 5); 32, 94 (each according to SEQ ID NO: 7); and 26 (according to SEQ ID NO:24).

16. A diagnostic primer as claimed in claim 15, which is an allele specific primer adapted for use in ARMS.

17. An allele-specific oligonucleotide probe capable of detecting a polymorphism in the KDR gene at one or more of positions: 224 (according to SEQ ID NO: 4); 145, 103 (each according to SEQ ID NO: 6); 162, 423, 461 (each according to SEQ ID NO: 1); 345 (according to SEQ ID NO: 2); 112, 329 (each according to SEQ ID NO: 3); 339 (according to SEQ ID NO: 5); 32, 94 (each according to SEQ ID NO: 7); and, 26 (according to SEQ ID NO:24).

18. An allele specific nucleotide probe which comprises the sequence disclosed in any one of SEQ ID Nos: 8 to 24, or a sequence complementary thereto.

19. A diagnostic kit comprising one or more diagnostic primer(s) as defined in claim 15 or 16 and/or one or more allele-specific oligonucleotide probes(s) as defined in claim 17 or 18.

20. A method of treating a human in need of treatment with a small molecule drug acting on the KDR (VEGFR2) protein or an anti-sense oligonucleotide or ribozyme acting against the KDR (VEGFR2) mRNA, in which the method comprises:
i) diagnosis of a polymorphism in the KDR gene in the human, which diagnosis preferably comprises determining the sequence at one or more positions selected from the group consisting of: position 224 (as defined by the position in SEQ ID NO: 4) and 145 (as defined by the position in SEQ ID NO: 6) in the coding region of the KDR; positions 162, 423 and 461 in the promoter region of the KDR gene (as defined by the position in SEQ ID NO: 1); gene; positions 345 (as defined by the position in SEQ ID NO: 2), 112, 329 (as defined by the position in SEQ ID NO: 3), 339 (as defined by the position in SEQ ID NO: 5), 103 (as defined by the position in SEQ ID NO: 6); 32, 94 (as defined by the position in SEQ ID NO: 7) in the intron region of the KDR gene; 26 in the 5' UTR (as defined by the position in SEQ ID NO: 24) in the exon 1 region of the KDR gene; and, amino acid residues 297, and 472 in the KDR polypeptide as defined by the position in EMBL Accession No: AF035121;
ii) determining the status of the human by reference to polymorphism in the KDR gene; and,
iii) administering an effective amount of the drug.

21. Use of a drug acting on the KDR (VEGFR2) protein or the KDR (VEGFR2) mRNA in preparation of a medicament for treating a disease in a human diagnosed as having a polymorphism therein, preferably at one or more of the positions identified in claim 3.

22. A pharmaceutical pack comprising a drug acting as a KDR antagonist and instructions for administration of the drug to humans diagnostically tested for a polymorphism therein, preferably at one or more of the positions identified in claim 3.

23. A computer readable medium having stored thereon a nucleic acid sequence comprising at least 17, preferably at least 20 consecutive bases of the KDR gene sequence, which sequence includes at least one of the polymorphisms at positions: 224 according to SEQ ID NO: 4; 145, 103according to SEQ ID NO:6; 162, 423, 461 according to SEQ ID NO: 1; 345 according to SEQ ID NO: 2; 112, 329 according to SEQ ID NO: 3; 339 according to SEQ ID NO:5; 32, 94 according to SEQ ID NO:7; or, 26 according to SEQ ID NO:24.
